# EUROPÄISCHE PATENTANMELDUNG

(11) **EP 1 588 701 A2**
(43) Veröffentlichungstag der Anmeldung: **26.10.2005**
(21) Anmeldenummer: 05010436.3
(22) Anmeldetag: 22.06.2001
(51) Int. Cl.: A61K 9/70

(54) **Schnell zerfallende Darreichungsform zur Freisetzung von Nicotin im Mundraum oder in Körperhöhlen**

(30) Priorität: 04.07.2000 DE 10032456
(62) Teilanmeldung aus: 01945296.0
(71) Anmelder: LTS LOHMANN Therapie-Systeme AG, 56626 Andernach (DE)
(72) Erfinder: von Falkenhausen, Christian, 53340 Meckenheim (DE); Krumme, Marcus, Randolph, NJ 0869 (US); Laux, Wolfgang, 56682 Diez (DE)
(74) Vertreter: Flaccus, Rolf-Dieter

(57) **Zusammenfassung**

Eine insbesondere flächenförmige, in wässriger Umgebung rasch zerfallende oder auflösbare Darreichungsform zur schnellen Freisetzung des Wirkstoffs Nicotin in der Mundhöhle, in Körperöffnungen oder in Körperhöhlungen, wobei die Darreichungsform eine Matrix, die ein oder mehrere wasserlösliche Polymere als Grundsubstanzen sowie den Wirkstoff Nicotin enthält und wobei die Darreichungsform dadurch gekennzeichnet ist, daß sie mit Räumen oder Hohlräumen versehen ist, die sich in der polymeren Matrix befinden und deren Inhalt sich hinsichtlich des Aggregatzustandes von der Matrix unterscheidet.

## Beschreibung

Die Erfindung bezieht sich auf vorzugsweise flächenförmige, in wässriger Umgebung schnell zerfallende Darreichungsformen, insbesondere orale Darreichungsformen, mittels derer eine rasche Freisetzung von Nicotin in der Mundhöhle oder in anderen Körperöffnungen bzw. Körperhöhlungen ermöglicht wird, und die eine Matrix auf der Basis von wasserlöslichen Polymeren als Grundsubstanzen aufweisen. Insbesondere bezieht sich die Erfindung auf Darreichungsformen der genannten Art, welche in Form von Oblaten ("wafer") gestaltet sind. Ferner schließt die Erfindung Verfahren zur Herstellung solcher Darreichungsformen mit ein.

Pharmazeutische Darreichungsformen, z.B. Buccal- oder Sublingualtabletten, die Wirkstoffe im Mundraum freisetzen, welche dann über die Mundschleimhaut resorbiert werden, sind in vielerlei Hinsicht vorteilhaft. Sie erleichtern die orale Verabreichung von Medikamenten an gewisse Patienten, denen die Einnahme anderer oraler Arzneiformen - z.B. aufgrund von Schluckbeschwerden - Schwierigkeiten bereitet. Da die Resorption über die Mundschleimhaut und unter Umgehung der Magen-Darm-Passage erfolgt, ist ein rascher Wirkungseintritt und eine hohe Wirkstoffausnutzung gewährleistet. Die genannten Vorteile treffen auch auf vaginale, rektale und intra-nasale Applikationsformen zu.

Als orale Arzneiformen, welche die vorstehend genannten Eigenschaften aufweisen, kommen neben Sublingual- oder Buccaltabletten auch flächenförmige, oblatenartige Darreichungsformen (auch "Wafer" genannt) in Betracht. Diese zeichnen sich aufgrund ihrer geringen Schichtdicke und raschen Zerfallsfähigkeit oder Auflösbarkeit insbesondere zur raschen Freisetzung von Medikamenten und anderen Wirkstoffen im Mundraum. In der Regel sind solche oblatenartigen Arzneiformen aus filmbildenden, wasserlöslichen Polymeren, wie z.B. bestimmten Cellulosederivaten aufgebaut. Bei Kontakt mit Wasser bzw. Speichel werden die Polymere gelöst und die Arzneiform zerfällt, wobei die in ihr befindlichen Wirkstoffe freigesetzt werden. Der Eintritt und der zeitliche Verlauf der Wirkstofffreisetzung hängt in hohem Maße von der Dicke der Arzneiform (des "Wafers") ab; je dünner sie ist, desto schneller schreitet der Zerfall in wässriger Umgebung voran, da das Lösungsmittel um so schneller in die inneren Bereiche der Arzneiform vordringen kann. Andererseits müssen solche Arzneiformen ("Wafer") eine gewisse Dicke aufweisen, um ihre bestimmungsgemäße Funktion, nämlich die Abgabe von Wirkstoffe, erfüllen zu können. Folglich wird die Dicke solcher Darreichungsformen wesentlich durch die Art und Menge des Wirkstoffes bedingt, den sie enthalten und freisetzen sollen. Mit zunehmender Dicke wird der Zerfall bzw. die Auflösung des "Wafers" entsprechend verlangsamt.
Insbesondere dickere "Wafer", aber auch solche mit einer relativ geringen Dicke, neigen aufgrund ihrer flächigen, glatten Form und des verzögerten Zerfalls dazu, am Gaumen oder an anderen Schleimhautoberflächen des Mundraums anzuhaften und festzukleben. Dies ist einerseits durch die sich oberflächlich lösenden Polymerschichten bedingt, welche einen klebrigen und matschigen Film bilden.

Durch die Eigenschaft dieser Arzneiformen, am Gaumen und anderen Oberflächen der Mundschleimhaut festzukleben, kann bei der betreffenden Person bzw. beim Patienten eine unangenehme Empfindung hervorrufen werden, d.h. das durch diese "Wafer" erzeugte "mouthfeel" ist unangenehm oder störend und deshalb verbesserungsbedürftig.

Aus EP 0 450 141 B2 ist ein Trägermaterial zur Verabreichung von Arzneimitteln etc. bekannt, welches sich nach oraler Aufnahme beim Kontakt mit Speichel schnell auflöst. Es handelt sich dabei um einen porösen dehydratisierten skelettartigen Trägerstoff, insbesondere auf der Basis von Proteinen und Polysacchariden. Die durch Dehydratisierung erzeugten Hohlräume werden für die Einbringung von flüssigen aktiven Substanzen genutzt. Die beschriebenen Gelatine-Polysaccharid-Träger können auch in Form von Oblaten verwendet werden. Maßnahmen zur Verminderung der Anhaftungstendenz sind nicht vorgesehen; diese Gefahr besteht aber, da die dehydratisierten Trägerstoffe spätestens bei Kontakt mit Speichel rehydratisiert werden und dadurch eine klebrige Oberfläche erhalten.

Es war deshalb die Aufgabe der vorliegenden Erfindung, eine Darreichungsform, insbesondere eine orale Darreichungsform der vorstehend genannten Art bereitzustellen, welche die bekannten Vorteile von flächenförmigen, schnell zerfallenden Darreichungsformen aufweist und darüber hinaus eine verringerte Tendenz zum Anhaften oder Festhaften an die Mundschleimhaut hat und sich deshalb durch ein verbessertes "mouthfeel" auszeichnet.

Die Aufgabe wird erfindungsgemäß dadurch gelöst, daß eine Darreichungsform mit den im Oberbegriff des Anspruchs 1 genannten Merkmalen mit Räumen oder Hohlräumen versehen ist, die sich in der polymeren Matrix der Darreichungsform befinden und deren Inhalt sich hinsichtlich des Aggregatzustandes von der Matrix unterscheidet. Insbesondere wird darunter verstanden, daß die Räume oder Hohlräume gasförmige oder flüssige Inhalte enthalten, während die Polymermatrix selbst einen festen oder halbfesten Aggregatszustand aufweist. Die erfindungsgemäßen Darreichungsformen weisen also räumliche Bereiche mit unterschiedlichen Phasen auf; die genannten Räume oder Hohlräume stellen eine zweite Phase dar, die sich im Innern der Polymermatrix (erste Phase) befinden kann, sie kann sich jedoch auch bis zum äußeren Rand erstrecken.

Durch die erfindungsgemäßen Räume oder Hohlräume wird zum einen der Zutritt von Wasser bzw. Speichel oder anderen Körperflüssigkeiten in das Innere der Darreichungsform (z.B. Oblate, Wafer) erleichtert und somit die Auflösung der Darreichungsform und die Wirkstofffreisetzung beschleunigt, welches insbesondere bei dickeren Darreichungsformen (Oblaten, Wafer) einen Vorteil darstellt. Zum anderen ist die Wandstärke der genannten Räume oder Hohlräume gering, da sie beispielsweise verfestigte Blasen darstellen, so daß eine schnelle Auflösung oder Zerstörung dieser Hohlräume stattfindet. Dadurch wird die innere Struktur und infolgedessen auch die Oberfläche der Darreichungsform verändert, so daß die Oberfläche unregelmäßig wird. Beispielsweise erhält die Oberfläche dadurch eine gewellte Struktur oder Unebenheiten. Dadurch und durch die Steifigkeit des Produkts wird das Anhaften der Oblate an die Mundschleimhaut verhindert.
Aufgrund der verringerten Anhaftungstendenz zeichnen sich die erfindungsgemäßen Darreichungsformen durch ein verbessertes "mouthfeel" aus, welches letztendlich zu einer verbesserten Akzeptanz bei den Anwendern oder Patienten führt.

Die Verweildauer der erfindungsgemäßen Darreichungsformen am Applikationsort (z. B. Mundraum), bzw. die Zerfallszeit, liegt vorzugsweise im Bereich von 1 s bis 5 min, stärker bevorzugt im Bereich von 5 s bis 1 min, und am meisten bevorzugt im Bereich von 10 s bis 30 s.

Die Erfindung ist jedoch nicht auf orale Darreichungsformen, welche Wirkstoffe im Bereich der Mundhöhle freisetzen, beschränkt. Sie erstreckt sich vielmehr auch auf Darreichungsformen, die in andere Körperhöhlungen oder Körperöffnungen eingebracht werden und dort ihre Wirkstoffe freisetzen, beispielsweise rektale, vaginale oder intra-nasale Darreichungsformen. Der freigesetzte Wirkstoff wird entweder am Applikationsort, z. B. über die Mundschleimhaut, resorbiert, oder er wird weitertransportiert und an einem anderen Ort resorbiert (z. B. im Magen-Darm-Trakt nach Verschlucken des in der Mundhöhle freigesetzten Wirkstoffs).

Die genannten Räume oder Hohlräume der Darreichungsform können jeweils isoliert voneinander in der Polymer-Matrix vorliegen, vorzugsweise in Form von verfestigten Blasen. Gemäß einer anderen Ausführungsform ist vorgesehen, daß die genannten Räume oder Hohlräume miteinander in Verbindung stehen, wobei sie vorzugsweise ein zusammenhängendes, die Matrix durchdringendes Kanalsystem bilden.

Als Darreichungsformen, welche die erfindungsgemäßen Räume oder Hohlräume aufweisen, eignen sich vor allem Polymermaterialien, welche verfestigte Schäume darstellen.

Die genannten Räume oder Hohlräume sind bevorzugt mit Gas oder einem Gasgemisch, insbesondere Luft, gefüllt; daneben kann es aber auch vorteilhaft sein, wenn sie andere Gase oder Gasgemische enthalten. Gemäß einer weiteren Ausführungsform ist vorgesehen, daß die Räume oder Hohlräume mit einer Flüssigkeit oder einem Flüssigkeitsgemisch gefüllt sind (beispielsweise Öle), wobei diese Flüssigkeiten nicht mit dem Matrixmaterial mischbar sind und das Polymergerüst nicht auflösen. Die genannte Flüssigkeit bzw. das Flüssigkeitsgemisch kann außerdem einen oder mehrere pharmazeutische Wirkstoffe enthalten.

Vorzugsweise haben die genannten Räume oder Hohlräume einen Gesamt-Volumenanteil von 5 bis 98 %, bevorzugt von 50-80 %, bezogen auf das Gesamtvolumen der Darreichungsform. Auf diese Weise wird der beabsichtigte anhaftungsvermindernde Effekt bewirkt, ohne daß die Wirkstoffaufnahmekapazität der Darreichungsform zu stark eingeschränkt wird.
Ein weiterer wichtiger Parameter, welcher die Eigenschaften der erfindungsgemäßen Darreichungsformen beeinflußt, ist der Durchmesser der Hohlräume oder Blasen. Die Blasen oder Hohlräume werden vorzugsweise mit Hilfe einer Schaumaufschlagmaschine erzeugt. Somit kann der Durchmesser der Blasen in einem weiten Bereich, fast beliebig, eingestellt werden. So kann der Durchmesser der Blasen oder Hohlräume im Bereich von 0,01 bis 50 µm liegen; besonders bevorzugt sind Durchmesser zwischen 0,1 und 10 µm.

Die Hohlräume der erfindungsgemäßen Darreichungsformen sind vorzugsweise wirkstofffrei; allerdings kann es von Vorteil sein, wenn diese Hohlräume Hilfs- oder Zusatzstoffe enthalten, vorzugsweise Tenside oder gasbildende Stoffe.

Um die Anhaftungstendenz der Darreichungsformen weiter zu vermindern, kann zusätzlich von der Maßnahme Gebrauch gemacht werden, daß die Oberflächen der Darreichungsform uneben oder unregelmäßig geformt sind, vorzugsweise wellenförmig oder reliefartig oder mit einer strukturierten Oberfläche. Eine solche unregelmäßige Oberflächenstruktur kann beispielsweise durch die in die Polymermatrix eingebrachten blasenförmigen Hohlräume verursacht werden, und/oder durch eine nachfolgende besondere Trocknungs-Behandlung.

Die Matrix der erfindungsgemäßen Darreichungsformen enthält als Grundsubstanzen ein wasserlösliches Polymer, oder Mischungen solcher Polymere. Dabei werden bevorzugt synthetische oder teilsynthetische Polymere oder Biopolymere natürlichen Ursprungs verwendet, die filmbildend und wasserlöslich sind, und/oder die sich zur Schaumbildung eignen. Besonders geeignet sind Polymere, die vorzugsweise aus der Gruppe ausgewählt sind, welche Cellulosederivate, Polyvinylalkohol, Polyacrylate und Polyvinylpyrrolidon umfaßt.

Unter den Cellulosederivaten werden Hydroxypropylmethylcellulose, Carboxymethylcellulose, Hydroxypropylcellulose, Hydroxymethylcellulose und Methylcellulose besonders bevorzugt, sowie andere substituierte Cellulose-Derivate. Ebenfalls bevorzugt sind wasserlösliche Polysaccharide, die pflanzlichen, mikrobiellen oder synthetischen Ursprungs sind, insbesondere solche Polysaccharide, die keine Cellulosederivate sind, wie z. B. Pullulan, Xanthan, Alginate, Dextrane, Agar-Agar, Pektine und Carrageen; das letztgenannte wird besonders bevorzugt.
Ferner sind auch Proteine, vorzugsweise Gelatine oder andere gelbildende Proteine, sowie Proteinhydrolysate, geeignet. Zu den geeigneten Proteinhydrolysaten gehören, unter anderem, Caseinat, Molke und pflanzliche Proteine, Gelatine sowie (Hühner-)Eiweiß, und Mischungen davon. Bevorzugte Proteine sind Caseinate, die von sprühgetrockneten Milchprodukten abstammen.

Die erfindungsgemäßen Darreichungsformen sind vorzugsweise dünn, z.B. in Form einer Oblate gestaltet. Die Dicke der Darreichungsform beträgt vorzugsweise zwischen 0,1 bis 5 mm, besonders bevorzugt zwischen 0,5 bis 1 mm. Die untere Grenze für die Dicke der Darreichungsformen liegt bei ca. 50 µm.

Als Wirkstoffe kommen - ohne Einschränkung - therapeutisch wirksame Verbindungen in Frage. Diese können aus folgenden Gruppen stammen: Mittel zur Infektionsbehandlung; Virostatika; Analgetika wie Fentanyl, Sufentanil, Buprenorphin; Anesthetika; Anorectika; Wirkstoffe zur Behandlung von Arthritis und Asthma, wie Terbutalin; Anticonvulsiva; Antidepressiva; Antidiabetika; Antihistaminika; Antidiarrhoika; Mittel gegen Migräne, Juckreiz, Übelkeit und Brechreiz; Reise- bzw. Seekrankheit, wie Scopolamin und Ondansetron; Parkinsonmittel; Antipsychotika; Antipyretika, Spasmolytika, Anticholinergika, Mittel gegen Ulkus wie Ranitidine, Sympathomimetika; Kalziumkanalblocker wie Nifedipin; Betablocker; Beta-Agonisten wie Dobutamin; Antiarrythmika; Antihypertonika wie Atenolol; ACE-Hemmer wie Enalapril; Benzodiazepin-Agonisten wie Flumazenil; coronare, periphere und zerebrale Vasodilatatoren; Stimulation für das Zentralnervensystem; Hormone; Hypnotika; Immunsuppresiva; muskelrelaxierende Mittel; Parasympatholytika; Parasympathomimetika; Prostaglandine; Proteine, Peptide; Psychostimulantien; Sedativa; Tranqilizer.

Für die Verabreichung im Mund bzw. an die Mundschleimhaut kommen grundsätzlich alle Wirkstoffe in Betracht, die buccal und/oder gastrointestinal resorbiert werden können. Hierbei wird Nicotin besonders bevorzugt.

Der Wirkstoffgehalt pro Dosiereinheit beträgt bis zu 50 mg, vorzugsweise bis 30 mg, besonders bevorzugt bis 20 mg.

Weiterhin kommen als Wirkstoffe in Betracht:
Poliermittel, Schleifmittel (abrasiv) wie Titandioxid, Siliciumdioxid etc.; Natriumfluorid, Dicalciumphosphat; etherische Öle wie Anis-Öl, Fenchelöl, Eucalyptusöl, Pfefferminzöl, Krauseminzöl, Orangenöl, Salbeiöl, Thymianöl, Zitronenöl, etc.; Aromastoffe wie Campher, Cineol, Eucalyptol, Menthol, Pinen, Zimtaldehyd, Zimtsäure, etc.; Honig, Citronensäure, Vitamine, Antioxidantien, Sorbit.
Die erfindungsgemäßen Darreichungsformen sind somit auch für kosmetische Anwendungszwecke, sowie für Anwendungen im Bereich der Zahnpflege, Zahnreinigung, Mundhygiene oder Dentalhygiene geeignet.

Als Aromastoffe können ferner Vanille-Aroma, Orangen-Aroma, Orangen-Rahm-Aroma, Erdbeer-Aroma, Himbeer-Aroma oder Schokoladen-Aroma zugesetzt werden, jeweils einzeln oder in Kombination. Außerdem können ein oder mehrere Süßstoffe zugesetzt werden, wie Sucralose, Aspartam, Cyclamat, Saccharin und Acesulfam, sowie deren Salze.

Als Hilfsstoffe kommen, unter anderem, Stoffe aus der folgenden Gruppe in Betracht:
Carboxymethylcellulose, Gummi aräbicum, Methylcellulose, Pektine, modifizierte und nicht modifizierte Stärken, Gelatine, tierische und/oder pflanzliche Proteine, Hühnereiweiß, Alginate, Bridge oder Brij (ein Emulgator), Isopropanol, Benzylalkohol, Ethylacetat, Ethylcitrat, Octylgallat, 1,2-Propylenglycat, Magnesiumstearat, Stearinsäure, mikrokristalline Cellulose, Aerosil, Lecithin, Tween, Propylgallat, Amylogam.

Zusätzlich kann in dem Schaum ein Zucker (oder eine Mischung von Zuckern), oder ein anderes Kohlenhydrat-Material gelöst werden. Der Zucker oder das Kohlenhydrat erhöht die Masse, welche der Schaum nach dem Trocknen hat. Außerdem verleiht das Trocknen und die Kristallisation des Zuckers, oder eines anderen Kohlenhydrates, dem getrockneten Schaum eine zusätzliche Festigkeit und Stabilität. Der Zucker oder andere Kohlenhydrate können bei dem getrockneten Schaum eine süße Geschmacksempfindung bewirken oder auf sonstige Weise die organoleptischen Eigenschaften des Schaums verbessern. Beispiele für hierfür verwendbare Zucker sind, unter anderem, Maltose, Lactose, Saccharose, Dextrose (Glucose) und Trehalose, sowie Zuckeralkohole, wie z. B. Mannit, Sorbit, Xylit, Maltit, und dergleichen. Als Beispiele für andere Kohlenhydrate kommen Maltodextrin, Stärkezuckersirup (aus Mais), lösliche Stärken und dergleichen in Betracht.

Bei der Herstellung der erfindungsgemäßen Darreichungsformen können außerdem eine oder mehrere Säuren beigemischt werden, um dem Schaum eine angenehme saure Geschmacksnote zu verleihen. Beispiele für derartige Säuren schließen, unter anderem, Citronensäure, Milchsäure, Essigsäure, Benzoesäure, Propionsäure, Oxalsäure, Malonsäure, Bernsteinsäure, Äpfelsäure und Weinsäure mit ein. Der Zusatz von Säure(n) kann außerdem notwendig oder erwünscht sein, um den pH-Wert des Schaums abzusenken. Dies ist insbesondere dann erwünscht, wenn der in der Darreichungsform enthaltene Wirkstoff unter basischen Bedingungen relativ unlöslich sind, wie z. B. Ibuprofen, oder bei Wirkstoffen, die unter basischen Bedingungen nicht stabil sind.

Ferner können den erfindungsgemäßen Darreichungsformen, insbesondere Schäumen, Anfeuchtungs- bzw. Feuchthaltemittel zugesetzt werden, um die ästhetischen Eigenschaften des getrockneten Schaums zu verbessern und um die Brüchigkeit oder Sprödigkeit des getrockneten Schaums zu vermindern. Beispiele für solche Mittel sind, unter anderem, Glycerin, Propylenglykol und Polyglycerin-Ester. Ferner können vor oder nach dem Trocknen oberflächenaktive Stoffe hinzugefügt werden, um die Stabilität des Schaums vor oder nach dem Trocknen zu verbessern. Als Beispiele für geeignete oberflächenaktive Stoffe kommen, unter anderem, substituierte Sorbitanderivate in Betracht, insbesondere diejenigen aus der "Tween"-Serie (ICI).

Für die Herstellung der erfindungsgemäßen Darreichungsformen mit verringerter Anhaftungstendenz und verbessertem "mouthfeel" werden die folgenden Verfahren vorgeschlagen:

Es wird zunächst eine Lösung oder Dispersion hergestellt, welche mindestens ein wasserlösliches filmbildendes Polymer sowie mindestens einen Wirkstoff enthält. Diese Lösung oder Dispersion, welche auch eine konzentrierte Lösung oder viskose Masse sein kann, wird anschließend durch Eintragen von Gas oder Gasgemisch (z. B. Luft) aufgeschäumt. Dies kann mittels eines Dispergierwerks oder einer Schaumaufschlagmaschine geschehen, aber auch durch andere Methoden, z.B. mittels Ultraschall. Als Gase eignen sich insbesondere auch inerte Gase, wie Stickstoff, Kohlendioxid oder Helium, oder Mischungen davon.
Um die so erzeugten Schäume oder luftblasenhaltigen (bzw. gasblasenhaltigen) Massen zu stabilisieren, wird vor oder während der Schaumerzeugung ein schaumstabilisierendes Mittel zugesetzt. Hierfür geeignete Mittel, z.B. Tenside, sind dem Fachmann bekannt. Schließlich wird die luftblasenhaltige Masse oder der Schaum auf einer geeigneten Unterlage als Film oder Schicht ausgestrichen und nachfolgend getrocknet. Durch Lösemittel-Entzug verfestigt sich der Schaum während der Trocknung zu einem Aerogel, wobei die gebildeten Hohlräume eine dauerhafte Struktur erhalten. Oblaten oder Wafer mit gewünschten Flächenausmaßen oder geometrischen Formen werden erhalten, indem die geschäumte Beschichtungsmasse in entsprechende Formen gegossen wird, oder indem die einzelnen Oblaten aus einem größeren Flächenstück ausgestanzt werden.
Die so erhaltenen wirkstoffhaltigen Arzneiformen weisen die erfindungsgemäßen Eigenschaften und Vorzüge auf.
Die Form, Anzahl und Größe der erzeugten Räume oder Hohlräume läßt sich mittels verschiedener Verfahrensparameter beeinflussen, z.B. durch die Art und Konzentration der Polymere, durch die viskosität der Polymermasse, durch Steuerung des Aufschäumungsvorganges, durch Auswahl der schaumstabilisierenden Mittel etc..

Bei einem anderen erfindungsgemäßen Verfahren zur Herstellung der genannten Darreichungsformen ist in Abwandlung zum vorstehend beschriebenen Verfahren vorgesehen, daß die Ausbildung der Räume oder Hohlräume im Inneren der Polymermatrix durch Einbringen eines hydrophoben, mit dem für die Herstellung der genannten Lösung oder Dispersion verwendeten Lösungsmittel nicht mischbaren Lösungsmittels erfolgt. Dabei wird eine Emulsion erzeugt, welche das hydrophobe Lösungsmittel in Form fein verteilter Tröpfchen enthält. Durch die Entfernung der Lösungsmittel während der nachfolgenden Trocknung bleiben in der Polymer-Matrix tröpfchen- oder blasenförmige Hohlräume zurück. Bei einem Zwei-PhasenSystem muß das Lösungsmittel der inneren Phase zuerst entzogen werden.

Ferner kann in Abwandlung des oben beschriebenen Verfahrens die Erzeugung der genannten Hohlräume auf die Weise geschehen, daß der polymer- und wirkstoffhaltigen Lösung oder Dispersion Hilfsstoffe zugesetzt werden, die ein Gas oder Gase bilden, wodurch die Masse aufgeschäumt wird. Dieses Aufschäumen durch Gasentwicklung kann entweder während der Herstellung der Polymermasse oder während der Beschichtung dieser Masse auf die Unterlage erfolgen, oder erst während des nachfolgenden Trocknungsprozesses. Zur Gasbildung geeignete Stoffe oder Stoffgemische sind dem Fachmann bekannt. Das Aufschäumen kann ferner auch durch Entspannung eines vorher gelösten Gases bewirkt werden. Als Gas kann insbesondere ein inertes Gase, wie Stickstoff, Kohlendioxid oder Helium, oder eine Mischung davon verwendet werden.

Bei der Herstellung der erfindungsgemäßen Darreichungsformen kann alternativ auch von einer Schmelze des Matrix-Polymers bzw. Polymergemisches ausgegangen werden. Die Verarbeitung erfolgt grundsätzlich ähnlich wie bei im Stand der Technik bekannten Heißschmelz("hot melt")-Beschichtungsmassen.
In die genannte Polymerschmelze wird durch eine der vorstehend genannten Methoden Gas oder ein Gasgemisch eingetragen, um ein Aufschäumen der Schmelze zu bewirken. Anschließend wird die Schmelze auf eine geeignete Unterlage ausgestrichen oder extrudiert oder in eine Form gegossen, und dann abkühlen bzw. erstarren lassen. Eine Verarbeitung aus der Schmelze kommt nicht in Frage, wenn der vorgesehene Wirkstoff bei der Schmelztemperatur der Polymerschmelze instabil oder flüchtig ist. Falls erforderlich, können der Polymerschmelze Hilfsstoffe zur Herabsetzung des Schmelzpunktes beigefügt werden. Grundsätzlich können auch aus dem Stand der Technik bekannte Heißschmelz-Beschichtungsmassen verwendet werden, sofern sie die in Anspruch 1 genannten Bedingungen erfüllen.

Gemäß einer weiteren Abwandlung der vorstehend beschriebenen Herstellungsverfahren wird die Polymermatrix zunächst in Form eines Blockes hergestellt. Von diesem werden nachfolgend, d. h. nach erfolgter Trocknung bzw. Erstarrung, durch Zerschneiden die gewünschten flächenförmigen Darreichungsformen abgetrennt.

Die erfindungsgemäßen Darreichungsformen eignen sich in vorteilhafter Weise für die Verabreichung von Medikamenten in der Mundhöhle oder zur rektalen, vaginalen oder intra-nasalen Verabreichung. Sie können in der Humanmedizin wie auch in der Veterinärmedizin eingesetzt werden.

### Herstellungsbeispiel

111,43 g destilliertes Wasser werden vorgelegt;
22,38 g Mowiol 8-88* werden unter Rühren zugegeben; Erhitzen des Ansatzes auf 80 °C;
Rühren (30 min);
Abkühlen auf 40 °C;
1,8 g PEG 400 werden hinzugefügt,
1,8 g PEG 4000 werden hinzugefügt;
Homogenisieren;
Zugabe von Aspartam (0,18 g) und Aroma (5,58 g);
Rühren;
Zugabe von 26,46 g Nikotinhydrogentartrat;
Zugabe von 1,8 g Siliciumdioxid;
Rühren (2 h) und Schaum einschlagen, bei Temperatur unterhalb von 50 °C;
Ausstreichen;
Trocknung bei 60°C (15 min).

* teilhydrolysierter Polyvinylalkohol niedriger Viskosität (Fa. Clariant)

## Patentansprüche

1. Insbesondere flächenförmige, in wässriger Umgebung rasch zerfallende oder auflösbare Darreichungsform zur schnellen Freisetzung des Wirkstoffs Nicotin in der Mundhöhle, in Körperöffnungen oder in Körperhöhlungen, wobei die Darreichungsform eine Matrix, die ein oder mehrere wasserlösliche Polymere als Grundsubstanzen sowie den Wirkstoff Nicotin enthält, **dadurch gekennzeichnet, daß** die Darreichungsform mit Räumen oder Hohlräumen versehen ist, die sich in der polymeren Matrix befinden und deren Inhalt sich hinsichtlich des Aggregatzustandes von der Matrix unterscheidet.

2. Darreichungsform nach Anspruch 1, **dadurch gekennzeichnet, daß** Nicotin in Form eines Salzes vorliegt, vorzugsweise als Nicotin-Bitartrat.

3. Darreichungsform nach Anspruch 1 oder 2, **dadurch gekennzeichnet, daß** die genannten Räume oder Hohlräume in der Matrix voneinander isoliert sind und vorzugsweise in Gestalt von Blasen vorliegen.

4. Darreichungsform nach Anspruch 1 oder 2, **dadurch gekennzeichnet, daß** die genannten Räume oder Hohlräume miteinander in Verbindung stehen, wobei sie vorzugsweise ein die Matrix durchdringendes Kanalsystem bilden.

5. Darreichungsform nach einem der Ansprüche 1 bis 4, **dadurch gekennzeichnet, daß** die genannten Räume oder Hohlräume mit einem Gas, Gasgemisch oder vorzugsweise mit Luft gefüllt sind.

6. Darreichungsform nach einem der Ansprüche 1 bis 4, **dadurch gekennzeichnet, daß** die genannten Räume oder Hohlräume mit einer Flüssigkeit oder einem Flüssigkeitsgemisch gefüllt sind, wobei die Flüssigkeit(en) nicht mit dem Matrixmaterial mischbar ist/sind.

7. Darreichungsform nach Anspruch 6, **dadurch gekennzeichnet, daß** die Flüssigkeit oder das Flüssigkeitsgemisch einen oder mehrere Wirkstoffe enthält.

8. Darreichungsform nach einem der Ansprüche 1 bis 7, **dadurch gekennzeichnet, daß** die genannten Räume oder Hohlräume einen Gesamt-Volumenanteil von 5 bis 98 %, bevorzugt von 50-80 % haben, bezogen auf das Gesamtvolumen der Darreichungsform.

9. Darreichungsform nach einem oder mehreren der Ansprüche 1 bis 8, **dadurch gekennzeichnet, daß** die genannten Räume oder Hohlräume der Darreichungsform im Zustand nach der Herstellung wirkstofffrei sind.

10. Darreichungsform nach einem oder mehreren der vorangehenden Ansprüche, **dadurch gekennzeichnet, daß** die Matrix der Darreichungsform einen verfestigten Schaum darstellt.

11. Darreichungsform nach einem oder mehreren der vorangehenden Ansprüche, **dadurch gekennzeichnet, daß** die Oberflächen der Darreichungsform uneben oder unregelmäßig geformt sind, vorzugsweise wellenförmig oder reliefartig.

12. Darreichungsform nach einem oder mehreren der vorangehenden Ansprüche, **dadurch gekennzeichnet, daß** der Polymeranteil der Matrix im wesentlichen aus einem oder mehreren synthetischen oder teilsynthetischen Polymer(en) oder natürlichen Biopolymeren besteht, welche filmbildend und wasserlöslich sind, wobei das/die Polymer(e) vorzugsweise aus der Gruppe ausgewählt wird/werden, die Cellulosederivate, Polyvinylalkohole, Polyacrylate und Polyvinylpyrrolidon umfaßt, wobei als Cellulosederivate Hydroxypropylmethylcellulose, Hydroxymethylcellulose, Carboxymethylcellulose, Hydroxypropylcellulose und Methylcellulose besonders bevorzugt werden, und die ferner wasserlösliche Polysaccharide pflanzlichen oder mikrobiellen Ursprungs, insbesondere Pullulan, Xanthan, Alginate, Dextrane, Agar-Agar, Pektine und Carrageen, sowie Proteine, vorzugsweise Gelatine, Caseinate oder andere gelbildende Proteine oder Proteinhydrolysate, umfaßt.

13. Darreichungsform nach einem oder mehreren der vorangehenden Ansprüche, **dadurch gekennzeichnet, daß** sie vorzugsweise als Oblate gestaltet ist, wobei die Dicke der Darreichungsform vorzugsweise zwischen 50 µm bis 5 mm, besonders bevorzugt zwischen 0,5 bis 1 mm beträgt.

14. Darreichungsform nach einem oder mehreren der vorangehenden Ansprüche, **dadurch gekennzeichnet, daß** die Matrix und/oder die genannten Räume oder Hohlräume Hilfs- oder Zusatzstoffe enthalten, vorzugsweise aus der Tenside oder gasbildende Stoffe, Anfeuchtungs- bzw. Feuchthaltemittel sowie schaumstabilisierende Stoffe umfassenden Gruppe.

15. Verfahren zur Herstellung einer flächenförmigen, in wässriger Umgebung rasch zerfallenden oder auflösbaren oralen Darreichungsform zur schnellen Freisetzung des Wirkstoffs Nicotin in der Mundhöhle, in Körperöffnungen oder in Körperhöhlungen, wobei die Darreichungsform eine Matrix, die ein oder mehrere Polymere als Grundsubstanzen sowie den Wirkstoff Nicotin enthält, und wobei die Darreichungsform mit Räumen oder Hohlräumen versehen ist, die sich in der polymeren Matrix befinden und deren Inhalt sich hinsichtlich des Aggregatzustandes von der Matrix unterscheidet, **gekennzeichnet durch** folgende Arbeitsschritte:
a) Herstellen einer Lösung oder Dispersion, welche mindestens ein wasserlösliches filmbildendes Polymer sowie den Wirkstoff Nicotin enthält;
b) Aufschäumen der Lösung oder Dispersion **durch** Eintragen von Gas oder Gasgemisch oder **durch** chemische Gaserzeugung oder **durch** Entspannen eines gelösten Gases, gegebenenfalls nach vorherigem Zusatz eines schaumstabilisierenden Mittels;
c) Ausstreichen der Lösung oder Dispersion auf eine Beschichtungsunterlage;
d) Verfestigung des hohlraumhaltigen Filmes **durch** Trocknen und Entzug des Lösemittels.

16. Verfahren zur Herstellung einer flächenförmigen, in wässriger Umgebung rasch zerfallenden oder auflösbaren oralen Darreichungsform zur schnellen Freisetzung des Wirkstoffs Nicotin in der Mundhöhle, in Körperöffnungen oder in Körperhöhlungen, wobei die Darreichungsform eine Matrix, die ein oder mehrere Polymere als Grundsubstanzen sowie den Wirkstoff Nicotin enthält, und wobei die Darreichungsform mit Räumen oder Hohlräumen versehen ist, die sich in der polymeren Matrix befinden und deren Inhalt sich hinsichtlich des Aggregatzustandes von der Matrix unterscheidet, **gekennzeichnet durch** folgende Arbeitsschritte:
a) Herstellen einer Lösung oder Dispersion, welche mindestens ein wasserlösliches filmbildendes Polymer sowie den Wirkstoff Nicotin enthält;
b) Zusatz eines hydrophoben, mit dem für die Herstellung der genannten Lösung oder Dispersion verwendeten Lösungsmittel nicht mischbaren Lösungsmittels, und Herstellen einer Emulsion, welche das hydrophobe Lösungsmittel in Form fein verteilter Tröpfchen enthält;
c) Ausstreichen der Lösung oder Dispersion auf eine Beschichtungsunterlage;
d) Verfestigung des Filmes unter Ausbildung von Hohlräumen **durch** Trocknen und Entzug der Lösemittel.

17. Verfahren zur Herstellung einer flächenförmigen, in wässriger Umgebung rasch zerfallenden oder auflösbaren oralen Darreichungsform zur schnellen Freisetzung des Wirkstoffs Nicotin in der Mundhöhle, in Körperöffnungen oder in Körperhöhlungen, wobei die Darreichungsform eine Matrix, die ein oder mehrere Polymere als Grundsubstanzen sowie den Wirkstoff Nicotin enthält, und wobei die Darreichungsform mit Räumen oder Hohlräumen versehen ist, die sich in der polymeren Matrix befinden und deren Inhalt sich hinsichtlich des Aggregatzustandes von der Matrix unterscheidet, **gekennzeichnet durch** folgende Arbeitsschritte:
a) Herstellen einer Lösung oder Dispersion, welche mindestens ein wasserlösliches filmbildendes Polymer sowie den Wirkstoff Nicotin enthält;
b) Zusatz eines Hilfsstoffes oder einer Kombination von Hilfsstoffen, welche zur Gasbildung befähigt sind;
c) Ausstreichen der Lösung oder Dispersion auf eine Beschichtungsunterlage;
d) Verfestigung des Filmes unter Ausbildung von Hohlräumen **durch** Trocknen und Lösemittel-Entzug.

18. Verfahren zur Herstellung einer flächenförmigen, in wässriger Umgebung rasch zerfallenden oder auflösbaren oralen Darreichungsform zur schnellen Freisetzung des Wirkstoffs Nicotin in der Mundhöhle, in Körperöffnungen oder in Körperhöhlungen, wobei die Darreichungsform eine Matrix, die ein oder mehrere Polymere als Grundsubstanzen sowie den Wirkstoff Nicotin enthält, und wobei die Darreichungsform mit Räumen oder Hohlräumen versehen ist, die sich in der polymeren Matrix befinden und deren Inhalt sich hinsichtlich des Aggregatzustandes von der Matrix unterscheidet, **gekennzeichnet durch** folgende Arbeitsschritte:
a) Herstellen einer polymerhaltigen Schmelze (hot melt), welche mindestens ein wasserlösliches filmbildendes Polymer sowie den Wirkstoff Nicotin enthält;
b) Aufschäumen der Schmelze **durch** Eintragen von Gas oder Gasgemisch oder **durch** chemische Gaserzeugung oder **durch** Entspannen eines gelösten Gases, gegebenenfalls nach vorherigem Zusatz eines schaumstabilisierenden Mittels;
c) Ausstreichen der Schmelze auf eine Beschichtungsunterlage;
d) Verfestigung des Filmes **durch** Abkühlung.

19. Verfahren nach einem der Ansprüche 15 bis 18, **dadurch gekennzeichnet, daß** die Schritte c) und d) durch folgende Schritte c) und d) ersetzt oder modifiziert werden:
c) Herstellen der Polymermatrix in Form eines Blockes, ausgehend von der Lösung oder Dispersion, bzw. von der Schmelze.
d) Zerschneiden des verfestigten Blocks, um flächenförmige Formen zu erhalten.

20. Verfahren nach einem der Ansprüche 15 bis 19, **dadurch gekennzeichnet, daß** Nicotin in Form eines Salzes eingesetzt wird, vorzugsweise als Nicotin-Bitartrat.

21. Verwendung einer Darreichungsform nach einem oder mehreren der Ansprüche 1 bis 14 oder eines Verfahrensprodukts aus einem der Verfahren 15 bis 20 zur Verabreichung von Nicotin in der Mundhöhle.

22. Verwendung einer Darreichungsform nach einem oder mehreren der Ansprüche 1 bis 14 oder eines Verfahrensprodukts aus einem der Verfahren 15 bis 20 zur rektalen, vaginalen oder intra-nasalen Verabreichung von Nicotin an Menschen oder Tiere.
